# EUROPEAN PATENT SPECIFICATION

(11) **EP 4 268 716 B1**
(45) Date of publication and mention of the grant of the patent: **03.09.2025**
(21) Application number: 21910442.9
(22) Date of filing: 13.12.2021
(51) Int. Cl.: A61B 5/05, B65D 81/38

(54) **INSULATED CONTAINER, AND MAGNETOENCEPHALOGRAPH AND MAGNETOSPINOGRAPH INCLUDING SAME**
ISOLIERTER BEHÄLTER UND MAGNETOENZEPHALOGRAPH UND MAGNETOSPINOGRAPH DAMIT
RÉCIPIENT ISOLÉ, ET MAGNÉTOENCÉPHALOGRAPHE ET MAGNÉTOSPINOGRAPHE LE COMPRENANT

(30) Priority: 23.12.2020 JP 2020213175
(43) Date of publication of application: 01.11.2023
(73) Proprietor: Arisawa Mfg. Co., Ltd., Joetsu-shi, Niigata 943-8610 (JP)
(72) Inventor: HIRAI, Masaaki, Joetsu-shi, Niigata 943-8610 (JP); NAKAMURA, Toshio, Joetsu-shi, Niigata 943-8610 (JP)
(74) Representative: V.O.
(86) International application number: PCT/JP2021/045874
(87) International publication number: WO 2022/138293

(56) References cited:
- JP-A- 2001 269 323
- JP-A- 2010 035 596
- JP-A- 2010 046 350
- JP-A- 2010 265 931
- JP-A- 2017 054 924
- JP-A- H0 323 683
- JP-A- H08 167 742
- JP-A- S6 254 982
- JP-A- S6 254 982
- US-A1- 2019 059 758

## Description

### Technical Field

The present disclosure relates to an insulated container, and a magnetoencephalograph and a magnetospinograph using the insulated container.

### Background Art

In the medical field, a device for measuring magnetic fields of the brain (magnetoencephalography (MEG)) has been put in practical use. The measurement device measures weak magnetic fields (one billionth of the earth magnetic field) generated in the brain, using a superconducting quantum interference device (SQUID) that makes use of Josephson effect.

Since the SQUID uses the superconductivity principle, the SQUID is required to be dipped in a refrigerant, such as liquid helium at -269°C (4.2 K). On this occasion, an insulated container that is capable of suppressing evaporation of liquid helium and storing liquid helium for a long time is required.

For example, in Patent Literature 1, an insulated container that is formed of fiber reinforced plastics (hereinafter, also referred to as "FRP") and includes an inner container, an outer container surrounding the inner container, and a support cylindrical body that supports the inner container is disclosed. On the inner surface and the outer surface of the inner container, a metal deposited layer is formed by vapor-depositing a metal. In addition, in the insulated container, a cavity between the inner container and the outer container is kept in a vacuum state. Since, in the insulated container, a metal deposited layer is formed on the inner surface and the outer surface of the inner container, helium gas is less likely to leak from the inner container when liquid helium is stored in the inner container. Thus, the insulated container can be kept in a vacuum state and good thermal insulation effect can be achieved.

### Citation List

### Patent Literature

Patent Literature 1: Unexamined Japanese Patent Application Publication No. H04-352404

Further relevant prior art is disclosed in JP 2017054924A, JP H0323683A, JP S62 54982A and JP H08167742A

### Summary of Invention

### Technical Problem

However, since heat is conducted from the outside to the inner container via the support cylindrical body, the insulated container does not have sufficient thermal insulation. Therefore, it is difficult to suppress evaporation of liquid helium stored in the inner container and is thus difficult to store liquid helium for a long time. Further, there is a problem in that the number of replenishments of liquid helium increases and the device cannot be operated in an uninterrupted manner.

The present disclosure has been made in consideration of the above-described circumstances, and an objective of the present disclosure is to provide an insulated container capable of storing a refrigerant for a long time and a magnetoencephalograph and a magnetospinograph using the insulated container.

### Solution to Problem

In order to achieve the above-described objective, [1] an insulated container according to the present disclosure includes an inner container and an outer container surrounding the inner container with a cavity interposed between the outer container and the inner container, in which the inner container and the outer container are formed of fiber reinforced plastics in which fibers are impregnated with resin, the inner container includes a bottomed cylindrical container to store a refrigerant and a refrigerant injection pipe attached to the cylindrical container and configured to inject a refrigerant into the cylindrical container, and fibers constituting the refrigerant injection pipe are wound in a spiral manner in an axial direction of the refrigerant injection pipe.

In addition, [2] fibers constituting the refrigerant injection pipe may be wound in a spiral manner in such a way as to form an angle of 50 to 89 degrees with an axial direction of the refrigerant injection pipe.

In addition, [3] fibers constituting the refrigerant injection pipe may be formed of at least one type of fiber selected from a group including glass fiber, alumina fiber, and carbon fiber.

In addition, [4] an amount of resin in fiber reinforced plastics that constitute the refrigerant injection pipe may be 15 to 40 wt% of entire weight of the fiber reinforced plastics.

In addition, [5] fibers constituting the cylindrical container may be formed of at least one type of fiber selected from a group including glass fiber, alumina fiber, and carbon fiber.

In addition, [6] the resin may include epoxy resin.

In addition, [7] a magnetoencephalograph according to the present disclosure includes a superconducting quantum interference device to detect a magnetic field, and the insulated container according to any one of [1] to [6], in which the superconducting quantum interference device is housed in the cylindrical container while being dipped in a refrigerant stored in the cylindrical container.

In addition, [8] the inner container may further include a second cylindrical container, each of the cylindrical container and the second cylindrical container may include a housing portion to store a refrigerant inside the housing portion, the second cylindrical container may be fixed to the cylindrical container while the housing portion of the second cylindrical container and the housing portion of the cylindrical container communicate with each other, and the second cylindrical container may be formed of fiber reinforced plastics in which fibers are impregnated with resin.

In addition, [9] fibers constituting the second cylindrical container may be formed of at least one type of fiber selected from a group including glass fiber, alumina fiber, and carbon fiber.

In addition, [10] resin constituting the second cylindrical container may include epoxy resin.

In addition, [11] a magnetospinograph according to the present disclosure includes a superconducting quantum interference device to detect a magnetic field, and the insulated container according to any one of [8] to [10], in which the superconducting quantum interference device is housed in the housing portion of the second cylindrical container while being dipped in a refrigerant stored in the housing portion of the second cylindrical container.

### Advantageous Effects of Invention

The present disclosure enables an insulated container capable of storing a refrigerant for a long time and a magnetoencephalograph and a magnetospinograph using the insulated container to be provided.

### Brief Description of Drawings

FIG. 1 is a schematic cross-sectional view of an insulated container according to Embodiment 1 of the present disclosure;
FIG. 2 is a schematic diagram describing orientation of fibers of fiber reinforced plastics constituting a refrigerant injection pipe illustrated in FIG. 1;
FIG. 3 is a schematic cross-sectional view of an insulated container according to Embodiment 2 of the present disclosure;
FIG. 4 is a schematic cross-sectional view of a magnetoencephalograph using the insulated container according to Embodiment 1 of the present disclosure;
FIG. 5 is a schematic cross-sectional view of a magnetospinograph using the insulated container according to Embodiment 2 of the present disclosure; and
FIG. 6 is a schematic cross-sectional view describing a wall constituting a cylindrical portion.

### Description of Embodiments

Embodiments for implementing the present disclosure (hereinafter, simply referred to as "embodiments") are described below in detail. The following embodiments are examples for describing the present disclosure and are not intended to limit the present disclosure only to the following description. The present disclosure can be appropriately modified and implemented without departing from the scope of the present disclosure.

### Embodiment 1

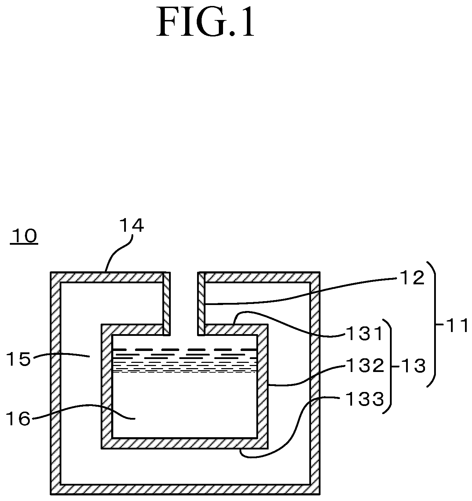

An insulated container 10 of Embodiment 1 is described. As illustrated in FIG. 1, the insulated container 10 is a container for storing an injected refrigerant 16. Specifically, the insulated container 10 includes an inner container 11 and an outer container 14 that surrounds the inner container 11 with a cavity 15 interposed therebetween.

The inner container 11 and the outer container 14 are described.

### (Inner Container 11)

The inner container 11 includes a bottomed cylindrical container 13 to store the refrigerant 16 and a refrigerant injection pipe 12 to inject the refrigerant 16 into the cylindrical container 13. The refrigerant injection pipe 12 is attached to an upper face portion of the cylindrical container 13. The refrigerant injection pipe 12 may be attached to a cylindrical portion of the cylindrical container 13.

In this configuration, when the cylindrical container 13 is stood in such a manner that the longitudinal direction (height direction) of the cylindrical container 13 is parallel with the vertical direction, a cylindrical portion constituting a sidewall of the cylindrical container 13 is referred to as "cylindrical portion", a portion serving as an upper face is referred to as "upper face portion", and a portion serving as a bottom face is referred to as "bottom face portion". The same applies to the following embodiments.

The refrigerant injection pipe 12 and the cylindrical container 13, which constitute the inner container 11, are described in this order.

### (Refrigerant Injection Pipe 12)

The refrigerant injection pipe 12 is a pipe body for injecting the refrigerant 16 into the cylindrical container 13. Examples of the shape of the pipe body include, although not limited to, a columnar shape, an elliptical columnar shape, and a quadrangular prism shape.

A thickness of the wall of the refrigerant injection pipe 12 is preferably 2 to 30 mm from a viewpoint of thermal insulation properties and a viewpoint of causing strength to appear at room temperature and in a temperature range of -200°C or less. As used herein, the "temperature range of -200°C or less" is also referred to as "cryogenic region". In addition, the thickness of the wall of the refrigerant injection pipe 12 means the thickness of a pipe constituting the refrigerant injection pipe 12 in a plane orthogonal to the longitudinal direction (height direction) of the refrigerant injection pipe 12.

A length of the refrigerant injection pipe 12 is appropriately set in accordance with sizes of the outer container 14 and the inner container 11 and is, for example, 100 to 500 mm.

A diameter of the refrigerant injection pipe 12 is, although not limited to, 50 to 300 mm from a viewpoint of preventing evaporation of the refrigerant.

When the shape of the refrigerant injection pipe 12 is an elliptical columnar shape, the length of the major axis is 50 to 300 mm.

When the shape of the refrigerant injection pipe 12 is a quadrangular prism shape, the length of the diagonal of a cross section of the pipe body in a plane orthogonal to the longitudinal direction (height direction) of the refrigerant injection pipe 12 is 100 to 400 mm.

The refrigerant injection pipe 12 is formed of fiber reinforced plastics (hereinafter, also referred to as "FRP") in which fibers are impregnated with resin.

In this configuration, a cure state of the resin constituting the FRP is a state in which curing reaction has completely finished (hereinafter, also referred to as "C-stage state"). Unless specifically noted, the cure state of the FRP is the C-stage state. The same applies to the following embodiments.

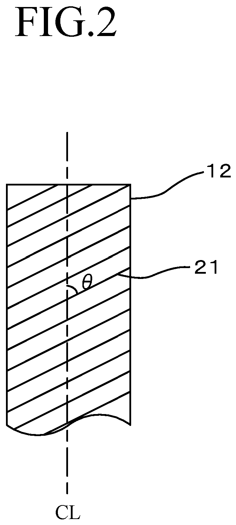

As illustrated in FIG. 2, fibers 21 of the FRP constituting the refrigerant injection pipe 12 are wound in a spiral manner in the extending direction of an axis CL of the refrigerant injection pipe 12 to slow conduction of heat from the outside to the inner container 11 via the refrigerant injection pipe 12 and are formed in a cylindrical shape. Specifically, the fibers 21 are wound in a spiral manner in such a way that the fibers 21 form an angle θ of 50 to 89 degrees with the extending direction of the axis CL of the refrigerant injection pipe 12. From a viewpoint of thermal conductivity and rigidity (elasticity) of the refrigerant injection pipe 12, a more preferable angle θ is 55 to 70 degrees.

The fibers 21 are formed of at least one type of fiber selected from a group including glass fiber, alumina fiber, and carbon fiber. The fibers 21 are preferably glass fibers from a viewpoint of price, processability, and availability of material and a viewpoint of reducing thermal conductivity in a temperature range from room temperature to the cryogenic region.

Examples of resin with which the fibers 21 are impregnated include, although not limited to, an epoxy-based resin composition containing an epoxy resin as a main component, a phenol-based resin composition containing a phenol resin as a main component, and a polyimide-based resin composition containing a thermosetting polyimide resin as a main component.

The amount of resin (in terms of solid content) with which the fibers 21 are impregnated is preferably 15 to 40 wt%, and more preferably 20 to 30 wt% of the entire weight of the FRP constituting the refrigerant injection pipe 12 from a viewpoint of preventing conduction of heat to the cylindrical container 13.

The amount of resin with which a base material, such as the fibers 21, is impregnated can be represented by the number of parts by weight or the amount of resin (wt%). The number of parts by weight is weight of resin when the weight of the base material is assumed to be 100 parts by weight. The amount of resin (wt%) is a parameter representing in ratio weight of resin adhering to a base material (fibers) with respect to the total weight of the base material and the resin and can be calculated by the formula: the amount of resin (wt%) = (weight of resin (wt) × 100)/(weight of fibers (wt) + weight of resin (wt)). In addition, unless specifically noted, all of the amounts of resin are the amount of resin in terms of solid content, and the amount of resin in terms of solid content means the amount of resin excluding a volatile component, such as a solvent.

The refrigerant injection pipe 12 can be produced by, for example, a filament winding method. A production method of the refrigerant injection pipe 12 is described later.

### (Cylindrical Container 13)

The cylindrical container 13 is a bottomed cylindrical container that stores the refrigerant 16. As illustrated in FIG. 1, the cylindrical container 13 includes an upper face portion 131 constituting the upper face, a cylindrical portion 132 constituting the sidewall, and a bottom face portion 133 constituting the bottom face. Examples of the shape of the cylindrical portion 132 include, although not limited to, a columnar shape, an elliptical columnar shape, and a quadrangular prism shape.

A thickness of the wall of the cylindrical container 13 is preferably 2 to 30 mm from a viewpoint of thermal insulation properties and a viewpoint of causing rigidity to appear in the cryogenic region. As used herein, the thickness of the wall of the cylindrical container 13 means the thickness of the wall constituting the cylindrical container 13 in a plane orthogonal to the longitudinal direction (height direction) of the cylindrical container 13.

The size of the cylindrical container 13 is, although not limited to, preferably a size that allows 50 to 150 liters of refrigerant to be stored when the insulated container 10 is used for, for example, a device for measuring a magnetic field of the head or the like of a human body.

The cylindrical container 13 is formed of FRP in which fibers are impregnated with resin.

Fibers constituting the FRP are formed of at least one type of fiber selected from a group including glass fiber, alumina fiber, and carbon fiber. The fibers constituting the FRP are preferably alumina fibers from a viewpoint of being nonmagnetic and reducing thermal conductivity in the cryogenic region.

For the fibers constituting the FRP, two types of fibers may be used in a mixed manner, and, for example, two types of fibers, namely alumina fiber and glass fiber, can be used.

Examples of a configuration of the fibers of the FRP constituting the cylindrical portion 132 of the cylindrical container 13 include, although not limited to, a configuration in which a plurality of fabrics each of which is a fabric into which fibers (warps and wefts) are woven is laminated in such a manner as to form a cylindrical shape and a configuration in which fibers are wound in a spiral manner. The configuration of the fibers of the FRP constituting the cylindrical portion 132 is preferably a configuration in which fibers are wound in a spiral manner from a viewpoint of increasing thermal insulation properties of the cylindrical container 13.

When the configuration in which a plurality of fabrics each of which is a fabric into which fibers are woven is laminated in such a manner as to form a cylindrical shape is employed, fabrics are laminated in such a manner that, for example, warps and wefts form an angle of 45 degrees and an angle of -45 degrees with the axial direction of the cylindrical portion 132, respectively. In addition, the fabrics may be laminated in such a manner that warps and wefts form an angle of 0 degrees and an angle of 90 degrees with the axial direction of the cylindrical portion 132, respectively. Examples of a method for weaving a fabric include a sateen weave, a plain weave, and a twill weave.

When the configuration in which fibers are wound in a spiral manner is employed, the fibers are wound in a spiral manner in such a way that the fibers form an angle of 50 to 89 degrees with the axial direction of the cylindrical portion 132.

Examples of the resin constituting the FRP include, although not limited to, an epoxy-based resin composition containing an epoxy resin as a main component, a phenol-based resin composition containing a phenol resin as a main component, and a polyimide-based resin composition containing a thermosetting polyimide resin as a main component. The resin constituting the FRP is preferably an epoxy-based resin composition from a viewpoint of having rigidity (elasticity) and toughness in a temperature range from room temperature to the cryogenic region and securing good processability. The resin may be a resin formed by mixing two or more types of resin compositions.

The amount of resin (wt%) of the cylindrical container 13 is preferably 15 to 40 wt%, and more preferably 20 to 30 wt%.

The amount of resin (the number of parts by weight) with respect to the fibers constituting the cylindrical container 13 is preferably 60 to 85 parts by weight, and more preferably 70 to 80 parts by weight when the weight of the entire fibers is assumed to be 100 parts by weight from a viewpoint of maintaining the rigidity of the cylindrical container 13 when storing refrigerant of -200°C or less.

The upper face portion 131 and the bottom face portion 133 of the cylindrical container 13 are formed of FRP. The configuration of the fibers of the FRP is a configuration in which a plurality of fabrics each of which is a fabric into which fibers are woven is laminated. The resin of the FRP is preferably the same resin as the resin of the cylindrical portion 132 and the refrigerant injection pipe 12 from a viewpoint of causing expansion and contraction behavior of the cylindrical container 13 and the refrigerant injection pipe 12 to be the same as expansion and contraction behavior of the upper face portion 131 and the bottom face portion 133 in a temperature range from room temperature to the cryogenic region.

Examples of the refrigerant 16 stored in the cylindrical container 13 include, although not limited to, liquid oxygen, liquid nitrogen, and liquid helium. In a use in the cryogenic region where a magnetic field of the head is measured, the refrigerant 16 is preferably liquid helium.

A production method of the cylindrical container 13 is described later.

### (Joining of Cylindrical Container 13 to Respective Members and Joining of Refrigerant Injection Pipe 12 to Cylindrical Container 13)

Examples of a joining method of the cylindrical container 13 to the upper face portion 131, a joining method of the cylindrical container 13 to the bottom face portion 133, and a joining method of the refrigerant injection pipe 12 to the cylindrical container 13 include, although not limited to, a method for joining joint portions by using an adhesive agent to the joint portions and a method for joining joint portions by forming a screw groove and a screw thread on joint surfaces of members and applying resin (adhesive agent) to the joint surfaces. The joining method is preferably a joining method that enables the cavity 15 to be kept in a vacuum state.

An adhesive agent to be used is preferably the same resin as the resin of which the refrigerant injection pipe 12 and the cylindrical container 13 are formed from a viewpoint of causing a good adhesiveness to appear on joint portions. Examples of the adhesive agent include an epoxy-based resin composition containing an epoxy resin as a main component, a phenol-based resin composition containing a phenol resin as a main component, and a polyimide-based resin composition containing a thermosetting polyimide resin as a main component. From a viewpoint of having rigidity (elasticity) and toughness in a temperature range from room temperature to the cryogenic region and securing good adhesiveness, the adhesive agent is preferably an epoxy-based resin composition. The adhesive agent may be formed by mixing two or more types of resin compositions.

### (Outer Container 14)

The outer container 14 is configured to surround the inner container 11 with the cavity 15 interposed therebetween, as illustrated in FIG. 1. Regarding the outer container 14, an upper face portion of the outer container 14 supports the cylindrical container 13 via the refrigerant injection pipe 12.

In this configuration, "to surround" means a state in which at least the outer container 14 surrounds the cylindrical container 13 in such a manner as to encase the cylindrical container 13 therein with the cavity 15 interposed therebetween.

A configuration in which the refrigerant injection pipe 12 and the cylindrical container 13 are surrounded by the outer container 14 is described below.

When the outer container 14 is stood in such a manner that the longitudinal direction (height direction) of the outer container 14 is parallel with the vertical direction, a portion serving as an upper face of the outer container 14 is referred to as "upper face portion", a cylindrical portion constituting a sidewall is referred to as "cylindrical portion", and a portion serving as a lower face is referred to as "bottom face portion". The "interior" of the outer container 14 means a region of the outer container 14 that is in contact with the cavity 15. The same applies to the following embodiments.

Between the outer container 14 and the cylindrical container 13, a not-illustrated support to support the cylindrical container 13 may be disposed in order to fix the cylindrical container 13 to a fixed position in the interior of the outer container 14.

The cavity 15 is preferably in a vacuum state from a viewpoint of increasing thermal insulation effect. The outer container 14 is configured to be strong enough to allow the shape of the outer container 14 to be retained even when the interior of the outer container 14 is brought into a vacuum state. The shape and the like of the outer container 14 are described below.

Examples of the shape of the outer container 14 include, although not limited to a specific shape as long as the shape surrounds the inner container 11, a columnar shape, an elliptical columnar shape, and a quadrangular prism shape. Among the above-described shapes, a columnar shape or an elliptical columnar shape is preferable from a viewpoint of preventing deformation of the container when the interior of the outer container 14 is brought into a vacuum state. Because of this configuration, it is possible to prevent contraction stress from concentrating on a specific portion of the container even when the cavity 15 is in a vacuum state.

The thickness of the wall of the outer container 14 is preferably the same as or thicker than the thickness of the cylindrical container 13 from a viewpoint of preventing deformation of the container when the interior of the outer container 14 is brought into a vacuum state. The thickness of the wall of the outer container 14 is, for example, 2 to 30 mm.

The size of the outer container 14 is only required to be, although not limited to, for example, a size large enough to surround the inner container 11.

The outer container 14 is formed of FRP in which fibers are impregnated with resin.

Fibers constituting the FRP are formed of at least one type of fiber selected from a group including glass fiber, alumina fiber, and carbon fiber. The fibers constituting the FRP are preferably glass fibers or alumina fibers from a viewpoint of strength retention and processability.

Example of the configuration of the fibers of the FRP include, although not limited to, the same configuration as the configuration of the cylindrical container 13.

Examples of the resin constituting the FRP include, although not limited to, an epoxy-based resin composition containing an epoxy resin as a main component, a phenol-based resin composition containing a phenol resin as a main component, and a polyimide-based resin composition containing a thermosetting polyimide resin as a main component. The resin constituting the FRP is preferably an epoxy-based resin composition from a viewpoint of having rigidity (elasticity) and toughness in a temperature range from room temperature to the cryogenic region and securing good processability. The resin may be formed by mixing two or more types of resin compositions.

The amount of resin (wt%) of the outer container 14 is preferably 15 to 40 wt%, and more preferably 20 to 30 wt% from a viewpoint of maintaining the rigidity of the cylindrical container 13 when storing refrigerant of -200°C or less.

The amount of resin (the number of parts by weight) with respect to the fibers constituting the outer container 14 is preferably 60 to 85 parts by weight, and more preferably 70 to 80 parts by weight when the weight of the entire fibers is assumed to be 100 parts by weight from a viewpoint of maintaining the rigidity of the cylindrical container 13 when storing refrigerant of -200°C or less.

A production method of the outer container 14 is described later.

### (Joining of Outer Container 14 to Respective Members and Joining of Outer Container 14 to Refrigerant Injection Pipe 12)

Examples of a joining method of the cylindrical portion to the upper face portion of the outer container 14, a joining method of the cylindrical portion to the bottom face portion of the outer container 14, and a joining method of the refrigerant injection pipe 12 to the upper face portion of the outer container 14 include, although not limited to, a method for joining joint portions by using an adhesive agent to the joint portions and a method for joining joint portions by forming a screw groove and a screw thread on joint surfaces of the members and applying resin (adhesive agent) to the joint surfaces. The joining method is preferably a joining method that enables the cavity 15 to be kept in a vacuum state.

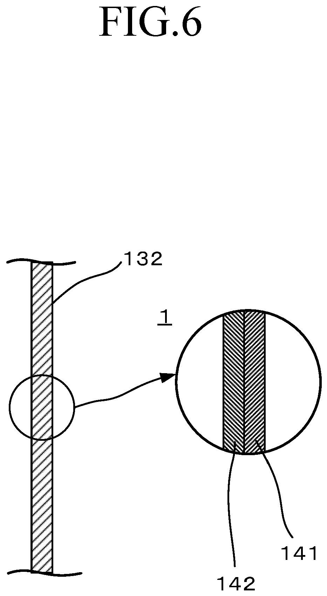

Examples of another configuration of the cylindrical container 13 include a cylindrical container 13 in which the wall of the cylindrical portion 132 has a two-layer structure. As illustrated in a circle 1 in FIG. 6, the wall of the cylindrical portion 132 includes an outer layer 141 and an inner layer 142. The outer layer 141 forms a layer closer to the cavity 15, and the inner layer 142 forms a layer closer to the refrigerant 16.

The outer layer 141 and the inner layer 142 are formed of FRP. Fibers constituting the FRP are glass fibers or alumina fibers.

Fibers constituting the outer layer 141 and fibers constituting the inner layer 142 are respectively wound in a spiral manner with respect to the extending direction of the axis of the cylindrical portion 132. Inclinations of the fibers with respect to the extending direction of the axis of the cylindrical portion 132 may be different from each other or the same as each other between the outer layer 141 and the inner layer 142. From a viewpoint of increasing the rigidity of the cylindrical portion 132 and improving workability (demoldability from a mold), an angle formed by the fibers constituting the inner layer 142 with respect to the extending direction of the axis is preferably larger than an angle formed by the fibers constituting the outer layer 141 with respect to the extending direction of the axis.

From a viewpoint of securing rigidity of the cylindrical portion 132 and slowing heat conduction from the outside to the entire inner container 11 via the refrigerant injection pipe 12, and reducing cost, the fibers constituting the outer layer 141 are preferably glass fibers. The fibers constituting the inner layer 142 are preferably alumina fibers.

From a viewpoint of slowing heat conduction from the outside to the entire inner container 11 via the refrigerant injection pipe 12 and preventing evaporation of a refrigerant, it is preferable that the fibers constituting the outer layer 141 be glass fibers, the fibers constituting the inner layer 142 be alumina fibers, and the outer layer 141 and the inner layer 142 have the same thickness or the inner layer 142 be thicker than the outer layer 141. The ratio of the thickness of the inner layer 142 to the thickness of the outer layer 141 is 50:50 to 80:20, preferably 50:50 to 70:30, and more preferably 50:50 to 60:40.

For resin with which the fibers constituting the outer layer 141 are impregnated and resin with which the fibers constituting the inner layer 142 are impregnated, the same resin is preferably used from a viewpoint of preventing delamination.

From a viewpoint of slowing heat conduction from the outside to the entire inner container 11 via the refrigerant injection pipe 12 and preventing evaporation of a refrigerant, when the configuration of the FRP of the cylindrical portion 132 is a two-layer structure, the configurations of the FRP of the upper face portion 131 and the bottom face portion 133 are also preferably a two-layer structure. In addition, fibers constituting the outer layer are preferably glass fibers, and fibers constituting the inner layer are preferably alumina fibers. In addition, it is preferable that the thicknesses of the outer layer and the inner layer be the same or the inner layer be thicker than the outer layer.

A production method in the case where the two-layer structure is employed is described in the production method of the cylindrical container 13.

Manufacturing methods of the refrigerant injection pipe 12, the cylindrical container 13, and the outer container 14 are described below.

### (Manufacturing Method of Refrigerant Injection Pipe 12)

The manufacturing method of the refrigerant injection pipe 12 includes, although not limited to, for example, a molding step of molding fibers into the shape of the refrigerant injection pipe 12 by winding fibers impregnated with resin around a rod-shaped mold in a spiral manner, a curing step of curing the resin through heating, and a demolding step of removing the mold from the cured refrigerant injection pipe 12.

Examples of a method for molding the shape of the refrigerant injection pipe 12 in the molding step include, although not limited to, a filament winding method. Fibers are wound around the mold in a spiral manner in such a way that an angle θ that the fibers form with the axial direction of the mold is 50 to 89 degrees.

Temperature and time required for curing the resin in the curing step can be appropriately set based on the type of resin, the amount of resin, and the like. For example, when an epoxy-based resin composition is used as the resin and the amount of resin (wt%) is set to 20 to 50 wt%, the temperature is 80 to 150°C and the time is 1 to 12 hours.

In the demolding step, the refrigerant injection pipe 12 that has been cooled to room temperature is removed from the mold, as a result of which the refrigerant injection pipe 12 is obtained.

### (Manufacturing Method of Cylindrical Container 13)

The cylindrical container 13 is, for example, obtained by, after individually producing the cylindrical portion 132, the upper face portion 131, and the bottom face portion 133, joining the cylindrical portion 132 and the upper face portion 131 to each other and joining the cylindrical portion 132 and the bottom face portion 133 to each other.

The cylindrical portion 132 is obtained by producing the cylindrical portion 132 using a similar method to the above-described manufacturing method of the refrigerant injection pipe 12.

The manufacturing method of the upper face portion 131 and the bottom face portion 133 includes, for example, a prepreg preparation step of producing a prepreg by impregnating a fabric into which fibers are woven with resin and subsequently heating the fabric, a cutting step of cutting the prepreg into predetermined shapes, and a curing step of laminating a plurality of cut prepregs and subsequently curing the laminated prepregs through heating and pressurization. In this processing, the prepreg means a composite material that is made of a fabric impregnated with resin and in which, after the fabric was impregnated with resin, the cure state of the resin is in a B-stage state. In addition, the B-stage state means a state in which curing reaction of the resin has not completely finished.

In the prepreg preparation step, temperature and time required for, after impregnating a fabric with resin, curing the resin to the B-stage state can be appropriately set based on the type of resin, the amount of resin adhesion (the amount of resin) to the fabric, and the like. For example, when an epoxy-based resin composition is used as the resin and the amount of resin (wt%) is set to 20 to 50 wt%, the temperature is 80 to 150°C and the time is 1 to 12 hours.

In the cutting step, the prepreg is cut into predetermined shapes in accordance with the shape of the cylindrical portion 132.

Temperature and time required for curing the prepregs in the curing step can be appropriately set based on the type of resin, the amount of resin, and the like. For example, when an epoxy-based resin composition is used as the resin and the amount of resin (wt%) is set to 20 to 50 wt%, the temperature is 80 to 150°C and the time is 1 to 12 hours.

Examples of a joining method of the upper face portion 131 to the cylindrical portion 132 and a joining method of the bottom face portion 133 to the cylindrical portion 132 include, although not limited to, for example, a method for joining the upper face portion 131 to the cylindrical portion 132 and the bottom face portion 133 to the cylindrical portion 132 by forming screw grooves and screw threads on joint portions of the upper face portion 131 and the cylindrical portion 132 and joint portions of the bottom face portion 133 and the cylindrical portion 132, respectively. In addition, the upper face portion 131 and the cylindrical portion 132 and the bottom face portion 133 and the cylindrical portion 132 may be joined to each other after applying an adhesive agent that is cured at room temperature to joint surfaces thereof on which screw grooves and screw threads are formed. This processing enables the joint portions to be securely sealed.

In addition, another manufacturing method of the cylindrical container 13 includes, for example, a prepreg preparation step of producing a prepreg by impregnating a fabric into which fibers are woven with resin and subsequently heating the fabric, a molding step of performing molding by sticking the prepreg on the surface of a mold copying the cylindrical container 13, a curing step of curing the prepreg through heating and pressurization, and a demolding step of removing the mold from the cured prepreg.

In the prepreg preparation step, temperature and time required for, after impregnating a fabric with resin, curing the resin to the B-stage state can be appropriately set based on the type of resin, the amount of resin adhesion (the amount of resin) to the fabric, and the like. For example, when an epoxy-based resin composition is used as the resin and the amount of resin (wt%) is set to 20 to 50 wt%, the temperature is 80 to 150°C and the time is 1 to 12 hours.

In the molding step, the molding is performed by, for example, sticking prepregs having been cut into predetermined sizes on the mold. When the shape of the mold is complex, the molding is performed by reducing the sizes of the prepregs and sticking the prepregs in accordance with the shape of the mold. This processing enables the prepregs to be molded in such a manner as to follow a more complex shape.

Examples of a method for curing the prepregs in the curing step include an autoclave molding method using an autoclave. Employing this method enables the prepregs to be cured under uniform conditions.

In the demolding step, the cylindrical container 13 is obtained by cooling the prepregs that were cured in the curing step, which is the preceding step, to room temperature and subsequently removing the prepregs from the mold.

In addition, when the configuration of the FRP of the cylindrical portion 132 is a two-layer structure, the cylindrical portion 132 having a two-layer structure can be produced by the filament winding method, which was described in the manufacturing method of the refrigerant injection pipe 12.

For example, when fibers constituting the inner layer are alumina fibers and fibers constituting the outer layer are glass fiber, the cylindrical portion 132 is produced as follows. Alumina fibers impregnated with resin are wound around a rod-shaped mold in a spiral manner. Next, by winding glass fibers impregnated with resin on the wound alumina fibers in a spiral manner, the shape of the cylindrical portion 132 is molded. Subsequently, the resin is cured through heating.

For the manufacturing method of the upper face portion 131 and the bottom face portion 133, the joining method of the upper face portion 131 to the cylindrical portion 132, and the joining method of the bottom face portion 133 to the cylindrical portion 132 when the configuration of the FRP of the cylindrical portion 132 is a two-layer structure, the same methods as the above-described manufacturing method of the upper face portion 131 and the bottom face portion 133, joining method of the upper face portion 131 to the cylindrical portion 132, and joining method of the bottom face portion 133 to the cylindrical portion 132 can be employed.

Note that the configuration of the upper face portion 131 and the bottom face portion 133 are preferably a two-layer structure including a layer formed of alumina fibers and a layer formed of glass fibers. In this case, the upper face portion 131 and the bottom face portion 133 are produced using a prepreg that is a fabric woven from alumina fibers and a prepreg that is a fabric woven from glass fibers.

### (Manufacturing Method of Outer Container 14)

Although the manufacturing method of the outer container 14 is not limited to specific one, the outer container 14 can be produced using, for example, a similar manufacturing method to the manufacturing method of the cylindrical container 13.

### Embodiment 2

An insulated container 40 of Embodiment 2 is described. In addition, constituent members common to Embodiment 1 are designated by the same reference numerals.

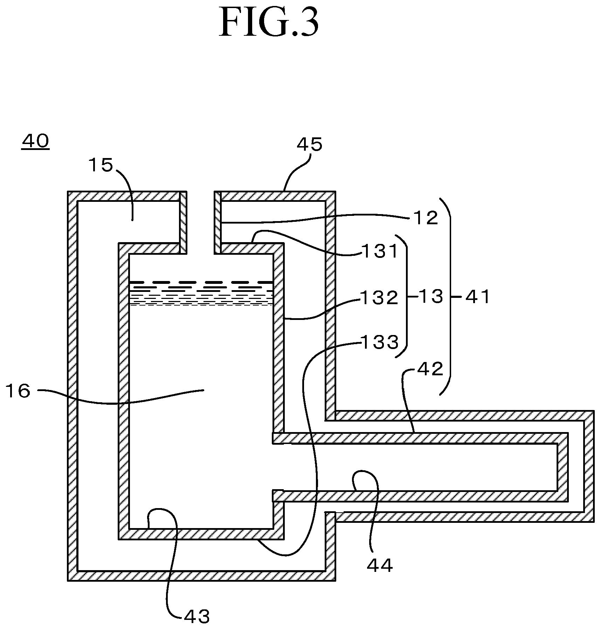

As illustrated in FIG. 3, the insulated container 40 is a container for storing a refrigerant 16. The insulated container 40 includes an inner container 41 and an outer container 45 that surrounds the inner container 41 with a cavity 15 interposed therebetween. In addition, the inner container 41 includes a cylindrical container 13 including a housing portion 43 to store the refrigerant 16 in the interior of the housing portion 43, a refrigerant injection pipe 12 attached to the cylindrical container 13, and a second cylindrical container 42 including a housing portion 44 to store the refrigerant 16 in the interior of the housing portion 44. In addition, the second cylindrical container 42 is fixed to the cylindrical container 13 while the housing portion 43 of the cylindrical container 13 and the housing portion 44 of the second cylindrical container 42 communicate with each other.

### (Inner Container 41)

The inner container 41 includes the cylindrical container 13 including the housing portion 43 to store the refrigerant 16 in the interior of the housing portion 43, the refrigerant injection pipe 12 attached to the cylindrical container 13, and the second cylindrical container 42 including the housing portion 44 to store the refrigerant 16 in the interior of the housing portion 44. In this configuration, the "interior" of the inner container 41 means a region that faces with which the refrigerant 16 has a possibility to come into contact form. The same applies to the following embodiment.

The housing portion 43 of the cylindrical container 13 and the housing portion 44 of the second cylindrical container 42 are configured while communicating with each other. The second cylindrical container 42 is fixed to the cylindrical container 13. Although the position at which the second cylindrical container 42 is fixed is not limited to specific one, the second cylindrical container 42 can be fixed to, for example, the cylindrical portion 132.

The housing portion 43 and the housing portion 44 may communicate with each other via a member such as a pipe. In this case, the member that communicate the housing portion 43 with the housing portion 44 is preferably formed of the same material as the material of which the cylindrical container 13 is formed.

### (Cylindrical Container 13)

The cylindrical container 13 includes the housing portion 43 to store the refrigerant 16 in the interior of the housing portion 43. The housing portion 43 is formed in the interior of the cylindrical container 13.

The cylindrical container 13 includes an upper face portion 131 constituting an upper face of the cylindrical container 13, a cylindrical portion 132 constituting a sidewall of the cylindrical container 13, and a bottom face portion 133 constituting a bottom face of the cylindrical container 13. Examples of the shape of the cylindrical portion 132 include, although not limited to, a columnar shape, an elliptical columnar shape, and a quadrangular prism shape.

### (Second Cylindrical Container 42)

The second cylindrical container 42 includes the housing portion 44 to store the refrigerant 16 in the interior of the housing portion 44. The housing portion 44 is formed in the interior of the second cylindrical container 42. The housing portion 44 communicates with the housing portion 43 of the cylindrical container 13. The second cylindrical container 42 is fixed to the cylindrical container 13.

In this configuration, when the second cylindrical container 42 is placed in such a manner that the longitudinal direction of the second cylindrical container 42 is orthogonal to the vertical direction, a cylindrical portion constituting the second cylindrical container 42 is referred to as "cylindrical portion" and a portion closing an opening formed at one end of the cylindrical portion is referred to as "bottom face portion". The same applies to the following embodiment.

The second cylindrical container 42 is fixed to the cylindrical portion 132 of the cylindrical container 13 in such a manner that the longitudinal direction of the second cylindrical container 42 is orthogonal to the vertical direction. Although a method for fixing the second cylindrical container 42 to the cylindrical container 13 is not limited to specific one, the second cylindrical container 42 can be fixed to the cylindrical container 13, using the joining method of members described in Embodiment 1.

Examples of the shape of the cylindrical portion of the second cylindrical container 42 include, although not limited to, a columnar shape, an elliptical columnar shape, and a quadrangular prism shape.

A thickness of the wall of the second cylindrical container 42 is, although not limited to, preferably 2 to 15 mm from a viewpoint of thermal insulation properties and a viewpoint of causing rigidity to appear in the cryogenic region. The thickness of the wall of the second cylindrical container 42 may be set to the same thickness as the thickness of the wall of the cylindrical container 13, depending on the intended use.

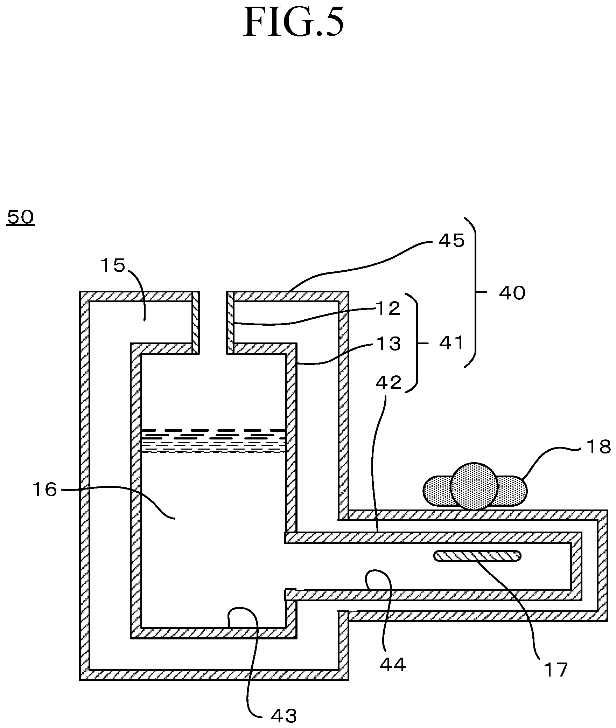

The size of the second cylindrical container 42 is preferably, although not limited to, a size that allows only a portion occupied by the second cylindrical container 42 to store 10 to 30 liters of refrigerant when the insulated container 40 is used for, for example, a device for measuring a magnetic field of the spinal cord or the like of a human body, as illustrated in FIG. 5.

The second cylindrical container 42 is formed of FRP in which fibers are impregnated with resin.

Fibers constituting the FRP are formed of at least one type of fiber selected from a group including glass fiber, alumina fiber, and carbon fiber. The fibers constituting the FRP are preferably alumina fibers from a viewpoint of being nonmagnetic and reducing thermal conductivity in the cryogenic region. The fibers constituting the FRP are more preferably fibers formed by mixing alumina fibers and glass fibers from a viewpoint of cost and productivity.

The cylindrical portion of the second cylindrical container 42 may have, although not limited to, for example, a configuration in which fibers are wound in a spiral manner with respect to the axial direction of the second cylindrical container 42 or a configuration in which a plurality of fabrics is laminated in such a manner as to form a cylindrical shape.

When fibers constituting the cylindrical portion of the second cylindrical container 42 are wound in a spiral manner, the fibers are wound in a spiral manner in such a way that the fibers form an angle of 50 to 89 degrees with the axial direction of the second cylindrical portion 42. From a viewpoint of making heat conducted from the outside via the refrigerant injection pipe 12 and the cylindrical container 13 less likely to be conducted to the entire second cylindrical container 42 and a viewpoint of increasing rigidity (elasticity) of the second cylindrical container 42, a more preferable angle is 55 to 70 degrees.

When the cylindrical portion of the second cylindrical container 42 has a configuration in which a plurality of fabrics constituting the cylindrical portion is laminated, it is preferable, although not limited thereto, that the plurality of fabrics be laminated in such a manner that, for example, warps constituting the fabrics form an angle of 0 degrees with the axial direction of the second cylindrical container 42 and wefts thereof form an angle of 90 degrees with the axial direction of the second cylindrical container 42 and thereby form a cylindrical shape. Note that the fabrics are preferably fabrics in plain weave or fabrics in twill weave from a viewpoint of rigidity, processability, and moldability and are preferably fabrics in sateen weave from a viewpoint of formability.

In addition, in order to prevent deformation of the container due to stress (expansion) caused by evaporation of the refrigerant 16, the amount of fiber oriented in the 90 degree direction (direction orthogonal to the axial direction) is preferably 10% or more greater than the amount of fiber oriented in the 0 degree direction (the axial direction).

The bottom face portion of the second cylindrical container 42 is formed of FRP. The fibers of the FRP have a configuration in which a plurality of fabrics is laminated. Resin constituting the FRP is preferably the same resin as the resin of the cylindrical container 13 from a viewpoint of causing the same expansion and contraction behavior of the bottom face portion of the second cylindrical container 42 as expansion and contraction behavior of the cylindrical container 13 to appear in a temperature range from room temperature to the cryogenic region.

Examples of the resin constituting the FRP include, although not limited to, an epoxy-based resin composition containing an epoxy resin as a main component, a phenol-based resin composition containing a phenol resin as a main component, and a polyimide-based resin composition containing a thermosetting polyimide resin as a main component. The resin constituting the FRP is preferably an epoxy-based resin composition from a viewpoint of having rigidity (elasticity) and toughness in a temperature range from room temperature to the cryogenic region and securing good processability. The resin may be a resin formed by mixing two or more types of resin compositions.

The amount of resin (wt%) of the second cylindrical container 42 is preferably 15 to 40 wt%, and more preferably 20 to 30 wt%.

The amount of resin (the number of parts by weight) with respect to fibers in the FRP is preferably 60 to 85 parts by weight, and more preferably 70 to 80 parts by weight when the weight of the entire fibers is assumed to be 100 parts by weight from a viewpoint of maintaining the rigidity of the cylindrical container 13 when storing refrigerant of -200°C or less.

### (Joining of Cylindrical Container 13 and Second Cylindrical Container 42)

Examples of a method for joining the cylindrical container 13 and the second cylindrical container 42 to each other include the joining method described in Embodiment 1.

A production method of the second cylindrical container 42 is described later.

### (Outer Container 45)

The outer container 45 is configured to surround the inner container 41 including the second cylindrical container 42 with the cavity 15 interposed therebetween, as illustrated in FIG. 3.

The shape and size of the outer container 45 are not limited to a specific shape and size as long as the outer container 45 can surround the inner container 41. From a viewpoint of preventing deformation of the container when the cavity 15 is brought into a vacuum state, the shape of the outer container 45 is preferably a rounded shape that enables stress to be distributed. In particular, the corner portions of the outer container 45 are preferably formed in shapes with large radii of curvature. Because of this configuration, it is possible to prevent contraction stress from concentrating on a specific portion of the container even when the cavity 15 is in a vacuum state.

A thickness of the wall of the outer container 45 is preferably the same as or thicker than the thickness of the cylindrical container 13 or the second cylindrical container 42 from a viewpoint of preventing deformation of the container when the cavity 15 is brought into a vacuum state. The thickness of the wall of the outer container 45 is, for example, 2 to 30 mm.

The manufacturing methods of the above-described cylindrical container 13, second cylindrical container 42, and outer container 45 are described below.

### (Manufacturing Method of Second Cylindrical Container 42)

The manufacturing method of the second cylindrical container 42 includes, for example, a prepreg preparation step of producing a prepreg by impregnating a fabric into which fibers are woven with resin and subsequently heating the fabric, a molding step of performing molding by sticking the prepreg on the surface of a mold copying the second cylindrical container 42, a curing step of curing the prepreg through heating and pressurization, and a demolding step of removing the mold from the cured prepreg.

In the prepreg preparation step, temperature and time required for, after impregnating a fabric with resin, curing the resin to the B-stage state can be appropriately set based on the type of resin, the amount of resin adhesion (the amount of resin) to the fabric, and the like. For example, when an epoxy-based resin composition is used as the resin and the amount of resin (wt%) is set to 20 to 50 wt%, the temperature is 80 to 150°C and the time is 1 to 12 hours.

In the molding step, the molding is performed by, for example, sticking prepregs having been cut into predetermined sizes on the mold. On this occasion, the fabrics are preferably laminated in such a manner that warps and wefts constituting the fabrics are oriented at an angle of 0 degrees and an angle of 90 degrees with respect to the axial direction of the mold, respectively, from a viewpoint of maintaining rigidity.

Examples of a method for curing the prepregs in the curing step include an autoclave molding method using an autoclave. Employing this method enables the prepregs to be cured under uniform conditions.

In the demolding step, the second cylindrical container 42 is obtained by cooling the prepregs that were cured in the curing step, which is the preceding step, to room temperature and subsequently removing the prepregs from the mold.

### (Manufacturing Method of Outer Container 45)

The manufacturing method of the outer container 45 includes, for example, a prepreg preparation step of producing a prepreg by impregnating a fabric into which fibers are woven with resin and subsequently heating the fabric, a molding step of performing molding by sticking the prepreg on the surface of a mold copying the outer container 45, a curing step of curing the prepreg through heating and pressurization, and a demolding step of removing the mold from the cured prepreg.

In the prepreg preparation step, temperature and time required for, after impregnating a fabric with resin, curing the resin to the B-stage state can be appropriately set based on the type of resin, the amount of resin adhesion (the amount of resin) to the fabric, and the like. For example, when an epoxy-based resin composition is used as the resin and the amount of resin (wt%) is set to 20 to 50 wt%, the temperature is 80 to 150°C and the time is 1 to 12 hours.

In the molding step, the molding is performed by, for example, sticking prepregs having been cut into predetermined sizes on the mold. When the shape of the mold is complex, the molding is performed by reducing the sizes of the prepregs and sticking the prepregs in accordance with the shape of the mold. This processing enables the prepreg to be molded in such a manner as to follow a more complex shape.

Examples of a method for curing the prepregs in the curing step include an autoclave molding method using an autoclave. Employing this method enables the prepregs to be cured under uniform conditions.

In the demolding step, the outer container 45 is obtained by cooling the prepregs that were cured in the curing step, which is the preceding step, to room temperature and subsequently removing the prepregs from the mold.

### Application Example 1

Application examples using the insulated container 10 according to Embodiment 1 include a magnetoencephalograph 30.

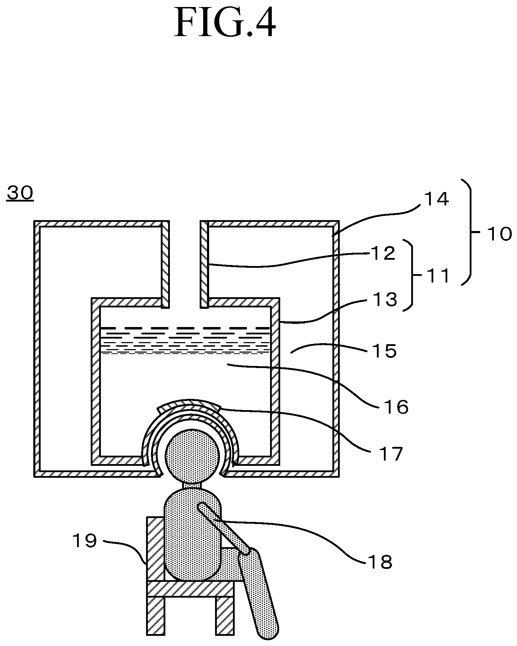

As illustrated in FIG. 4, the magnetoencephalograph 30 includes a superconducting quantum interference device (SQUID) 17 to detect a magnetic field generated from a living body 18 and an insulated container 10. The insulated container 10 includes an inner container 11 and an outer container 14 that surrounds the inner container 11 with a cavity interposed therebetween. The inner container 11 includes a bottomed cylindrical container 13 to store a refrigerant 16 and a refrigerant injection pipe 12 attached to the cylindrical container 13. The superconducting quantum interference device 17 is housed in the cylindrical container 13 while being dipped in the refrigerant 16 stored in the cylindrical container 13. The living body 18 sits on a chair 19 in FIG. 4.

### Application Example 2

Application examples using the insulated container 40 according to Embodiment 2 include a magnetospinograph 50.

As illustrated in FIG. 5, the magnetospinograph 50 includes a superconducting quantum interference device 17 to detect a magnetic field generated from a living body 18 and an insulated container 40. The insulated container 40 includes an inner container 41 and an outer container 45 that surrounds the inner container 41 with a cavity 15 interposed therebetween. The inner container 41 includes a cylindrical container 13 including a housing portion 43 to store a refrigerant 16 in the interior of the housing portion 43, a refrigerant injection pipe 12 attached to the cylindrical container 13, and a second cylindrical container 42 including a housing portion 44 to store the refrigerant 16 in the interior of the housing portion 44. The superconducting quantum interference device 17 is housed in the second cylindrical container 42 while being dipped in the refrigerant 16 stored in the second cylindrical container 42. The living body 18 lies down in such a manner that a spinal cord portion of the living body 18 is positioned over the superconducting quantum interference device 17 in FIG. 5.

### (Advantageous Effects)

The insulated container 10 according to Embodiment 1 described above and the magnetoencephalograph 30 using the insulated container 10 and the insulated container 40 according to Embodiment 2 described above and the magnetospinograph 50 using the insulated container 40 achieve the following advantageous effects when storing the refrigerant 16.

The fibers of the FRP constituting the refrigerant injection pipe 12 being wound in a spiral manner in the axial direction of the refrigerant injection pipe 12 and formed in a cylindrical shape causes conduction of heat from the outside to the inner container 11 via the refrigerant injection pipe 12 to be slowed. That is, since heat from the outside is conducted along the fibers wound in a spiral manner, conduction of heat becomes slower than in the case of a refrigerant injection pipe on which fibers are oriented linearly in the same direction as the axial direction (0 degrees), as a result of which thermal insulation properties are improved.

As a result, in the insulated container of each of the embodiments, conduction of heat reaching the cylindrical container via the refrigerant injection pipe is slowed and the insulated container becomes less likely to warm. That is, it becomes possible to slow the evaporation speed of a refrigerant.

Further, since, in the magnetoencephalograph and the magnetospinograph using the insulated containers of the embodiments, the evaporation speed of a refrigerant is slowed, it is possible to reduce the number of replenishments of the refrigerant, which enables long-time uninterrupted operation of the magnetoencephalograph and the magnetospinograph.

### Examples

Thermal insulation effect was confirmed using the following Examples and Comparative Examples. Note that the present disclosure is not limited by the following Examples at all.

Resins and agents used in Examples and Comparative Examples are as follows.

### (Resin)

### (1) Matrix resin A:

(1-1) base resin: bisphenol A-type epoxy resin (manufactured by The Dow Chemical Company, DER383LCL),
(1-2) curing agent: acid anhydride-based curing agent (manufactured by Hitachi Chemical Company, Ltd., HN-2000), and
(1-3) curing accelerator: N, N-dimethyl benzyl amine (manufactured by Kao Corporation, Kaolyzer No. 20).

### (2) Matrix resin B:

(2-1) base resin: bisphenol A-type epoxy resin (manufactured by DIC Corporation, EPICLON850),
(2-2) curing agent: amine-based curing agent (manufactured by Nippon Carbide Industries Co., Inc., DICY), and
(2-3) curing accelerator: dimethyl benzyl amine (manufactured by Kao Corporation, Kaolyzer No. 20).

### (Base Member)

Fibers and fabrics used in Examples and Comparative Examples are as follows:
(1) glass fiber (manufactured by Jushi Group. Co., Ltd., EDR24-2400-318),
(2) alumina fiber (manufactured by 3M Company, XN-508),
(3) alumina cloth (manufactured by Marukatsu Co., Ltd., alumina cloth),
(4) glass cloth A (manufactured by Arisawa Fiber Glass Co., Ltd., K7628), and
(5) glass cloth B (manufactured by Arisawa Fiber Glass Co., Ltd., ECC181).

In Examples and Comparative Examples, respective evaluation methods and measurement methods were performed by the following methods.

### (Measurement of Thermal Conductivity)

With respect to refrigerant injection pipes and cylindrical containers, thermal conductivities in the axial direction (longitudinal direction) were measured. The thermal conductivities (W/m·K) were measured using a measurement device (manufactured by AGNE Gijutsu Center Inc., ARC-TC-1000). As a measurement method, a temperature gradient method was employed. Measurement conditions conformed to ASTME1225.

### (Measurement of Evaporation Amount of Gas)

A produced insulated container (inner container) was filled with liquid helium (manufactured by Japan Helium Center Corporation) and the amount of helium gas that evaporated and came out of the container was measured by a gas flowmeter (manufactured by TOKYOSHINAGAWA, WSDa-2.5A). A measurement method conformed to JIS B7550.

Evaluation was performed using the following criteria:
Excellent: 8.0 L/day or less;
Good: 8.1 L/day or more and less than 9.0 L/day; and
Poor: 9.0 L/day or more.

### (Refrigerant Injection Pipe)

In order to perform evaluation of thermal conductivity of refrigerant injection pipes, refrigerant injection pipes in Examples 1 to 4 and Comparative Examples 1 to 3 were produced. Table 1 illustrates configurations of the refrigerant injection pipes and results of thermal conductivity measurements.

### Example 1

### (1) Preparation of Resin for Refrigerant Injection Pipe

Resin for a refrigerant injection pipe was prepared using the base resin, the curing agent, and the curing accelerator of the matrix resin A. First, 100 parts by weight of base resin, 88 parts by weight of curing agent, and 0.8 parts by weight of curing accelerator were added into a container. Subsequently, the mixture of the base resin, the curing agent, and the curing accelerator was stirred by a high-speed mixer under the condition of room temperature, 700 rpm, and 60 minutes, as a result of which the matrix resin A was obtained. Viscosity at that time was 500 mPa·s. Note that methyl ethyl ketone (MEK) was appropriately added as an organic solvent in such a manner that the viscosity of the matrix resin A became a predetermined viscosity.

### (2) Production of Refrigerant Injection Pipe

The glass fibers were impregnated with the matrix resin A in such a manner that the amount of resin (wt%) of a refrigerant injection pipe after curing becomes 25 wt%. The glass fibers impregnated with the matrix resin A were wound around a rod made of stainless steel with a diameter of 150 mm and a length of 2000 mm in a spiral manner in such a way that the glass fibers formed an angle of 55 degrees with the axial direction of the rod. Subsequently, a polyethylene film was wrapped on the glass fibers wound in a spiral manner and a polypropylene film was further wrapped on the polyethylene film. The glass fibers in this state were heated at 130°C for 8 hours. After the heating, the glass fibers were cooled to room temperature and the rod made of stainless steel was removed, as a result of which the refrigerant injection pipe with a wall thickness of 5 mm and a length of 300 mm was obtained.

### Comparative Example 1

### (1) Preparation of Resin for Refrigerant Injection Pipe

Resin for a refrigerant injection pipe was prepared using the base resin, the curing agent, and the curing accelerator of the matrix resin B. First, 100 parts by weight of base resin, 4 parts by weight of curing agent, and 0.2 parts by weight of curing accelerator were added into a container. Subsequently, the mixture of the base resin, the curing agent, and the curing accelerator was stirred by a high-speed mixer under the condition of room temperature, 700 rpm, and 60 minutes, as a result of which the matrix resin B was obtained. Viscosity at that time was 100 mPa·s. Note that methyl ethyl ketone (MEK) was appropriately added as an organic solvent in such a manner that the viscosity of the matrix resin B became a predetermined viscosity.

### (2) Production of Refrigerant Injection Pipe

The glass cloth A was impregnated with the matrix resin B in such a manner that the amount of resin (wt%) of a refrigerant injection pipe after curing becomes 25 wt%. The glass cloth A impregnated with the matrix resin B was wrapped around a rod made of stainless steel with a diameter of 150 mm and a length of 1000 mm in such a manner that warps and wefts of the glass cloth A formed an angle of 0 degrees and an angle of 90 degrees with the axial direction of the rod, respectively. Subsequently, a polypropylene film was wrapped on the wrapped glass cloth A. The glass cloth A in this state was heated at 130°C for 2 hours. Subsequently, the glass fibers were cooled to room temperature and the rod made of stainless steel was removed, as a result of which the refrigerant injection pipe was obtained.

### Examples 2 to 4 and Comparative Examples 2 and 3

As illustrated in Table 1, refrigerant injection pipes were produced in the same manner as in Example 1 or Comparative Example 1 except that base materials, angles, and matrix resins were changed.

**[Table 1]**

| | Example 1 | Example 2 | Example 3 | Example 4 | Comparative Example 1 | Comparative Example 2 | Comparative Example 3 |
|---|---|---|---|---|---|---|---|
| Base Material | Glass Fiber | Glass Fiber | Alumina Fiber | Alumina Fiber | Glass Cloth A | Glass Cloth A | Alumina Cloth |
| Angle (degree) | 55 | 70 | 50 | 70 | 0, 90 | 0, 90 | 0, 90 |
| Matrix Resin | A | A | A | A | B | A | B |
| Axial Direction Thermal Conductivity (25°C) [w/m·k] | 0.47 | 0.46 | 0.62 | 0.63 | 0.52 | 0.52 | 0.69 |

### (Cylindrical Container)

In order to perform evaluation of thermal conductivity of cylindrical containers, cylindrical containers in Examples 5 to 11 were produced. Table 2 illustrates configurations of the cylindrical containers and results of thermal conductivity measurements.

### Example 5

### (1) Preparation of Resin for Cylindrical Container

In the same manner as in Example 1, resin for a cylindrical container was prepared using the base resin, the curing agent, and the curing accelerator of the matrix resin A. First, 100 parts by weight of base resin, 88 parts by weight of curing agent, and 0.8 parts by weight of curing accelerator were added into a container. Subsequently, the mixture of the base resin, the curing agent, and the curing accelerator was stirred by a high-speed mixer under the condition of room temperature, 700 rpm, and 60 minutes, as a result of which the matrix resin A was obtained. Viscosity at that time was 500 mPa·s. Note that methyl ethyl ketone (MEK) was appropriately added as an organic solvent in such a manner that the viscosity of the matrix resin A became a predetermined viscosity.

### (2) Production of Cylindrical Container

### (2-1) Production of Cylindrical Portion

The glass fibers were impregnated with the matrix resin A in such a manner that the amount of resin (wt%) of a cylindrical container after curing becomes 25 wt%. The glass fibers impregnated with the matrix resin A were wound around a rod made of stainless steel with a diameter of 415 mm and a length of 2000 mm in a spiral manner in such a way that the glass fibers formed an angle of 55 degrees with the axial direction of the rod. Subsequently, a polyethylene film was wrapped on the glass fibers wound in a spiral manner and a polypropylene film was further wrapped on the polyethylene film. The glass fibers in this state were heated at 130°C for 8 hours. After heating, the glass fibers were cooled to room temperature and the rod made of stainless steel was removed, as a result of which the cylindrical portion was obtained.

### (2-2) Production of Upper Face Portion and Bottom Face Portion of Cylindrical Container

Glass fibers were woven in plain weave using an air-jet loom (manufactured by Toyota Industries Corporation, JAT810), as a result of which the glass cloth A was obtained. The mass per unit area of the glass cloth A was 210 g/m². Next, the same resin as the resin for a cylindrical container used in Comparative Example 1 (the matrix resin B) was prepared. After the glass cloth A was impregnated with the prepared resin, prepregs were produced in such a manner that the amount of resin (wt%) after curing under the drying condition of 100°C and 10 minutes became 30 wt%.

A plurality of prepregs was laminated in such a manner that the thickness of a laminated plate after curing becomes 30 mm and was heated and pressurized under the condition of 130°C, 0.5 MPa, and 2 hours, as a result of which the laminated plate was obtained. The laminated plate was cut in accordance with the sizes of the upper face and bottom face of the cylindrical container, as a result of which the upper face portion and the bottom face portion were obtained.

### (2-3) Joining of Upper Face Portion and Bottom Face Portion to Cylindrical Portion

The upper face portion and the bottom face portion were joined to the cylindrical portion. At the time of joining, screw grooves were formed on the upper end side and the lower end side of the inner wall of the cylindrical portion. Further, screw threads corresponding to the screw grooves were formed on the side surfaces of the upper face portion and the bottom face portion (at locations coming into contact with the screw grooves on the cylindrical portion).

After an adhesive agent (room temperature curing epoxy resin, manufactured by Henkel Japan Ltd., Stycast 1266) was applied to joint surfaces of the cylindrical portion, the upper face portion, and the bottom face portion, the cylindrical portion and the upper face portion were joined to each other and the cylindrical portion and the bottom face portion were joined to each other. In addition, joints were sealed using an adhesive agent (room temperature curing epoxy resin, manufactured by Henkel Japan Ltd., Stycast 1266). After drying, the cylindrical container was obtained.

### Example 9

### (1) Preparation of Resin for Cylindrical Container

Resin for a cylindrical container was prepared using the base resin, the curing agent, and the curing accelerator of the matrix resin B. First, 100 parts by weight of base resin, 4 parts by weight of curing agent, and 0.2 parts by weight of curing accelerator were added into a container. Subsequently, the mixture of the base resin, the curing agent, and the curing accelerator was stirred by a high-speed mixer under the condition of room temperature, 700 rpm, and 60 minutes, as a result of which the matrix resin B was obtained. Viscosity at that time was 100 mPa·s. Note that methyl ethyl ketone (MEK) was appropriately added as an organic solvent in such a manner that the viscosity of the matrix resin B became a predetermined viscosity.

### (2) Production of Cylindrical Container

The glass cloth A was impregnated with the matrix resin B in such a manner that the amount of resin (wt%) of a cylindrical container after curing becomes 30 wt%. The glass cloth A impregnated with the matrix resin B was wrapped around a rod made of stainless steel with a diameter of 415 mm and a length of 1000 mm in such a manner that warps and wefts of the glass cloth A formed an angle of 0 degrees and an angle of 90 degrees with the axial direction of the rod, respectively. Subsequently, a polypropylene film was wrapped on the wrapped glass cloth A. The glass cloth A in this state was heated at 130°C for 2 hours. Subsequently, the glass cloth A were cooled to room temperature and the rod made of stainless steel was removed, as a result of which the cylindrical container was obtained.

As the upper face portion and bottom face portion of the cylindrical container in Example 9, the same upper face portion and bottom face portion as the upper face portion and the bottom face portion in Example 5 were produced and used, respectively. In addition, the upper face portion and the bottom face portion were joined to the cylindrical portion using the same joining method as that in Example 5 and the joined cylindrical portion, upper face portion, and bottom face portion were dried, as a result of which the cylindrical container was obtained.

### Examples 6 to 8, 10, and 11

As illustrated in Table 2, cylindrical containers were produced by the same method as that in Example 5 or 9 except that base materials, angles, and matrix resins were changed.

**[Table 2]**

| | Example 5 | Example 6 | Example 7 | Example 8 | Example 9 | Example 10 | Example 11 |
|---|---|---|---|---|---|---|---|
| Base Material | Glass Fiber | Glass Fiber | Alumina Fiber | Alumina Fiber | Glass Cloth A | Glass Cloth A | Alumina Cloth |
| Angle (degree) | 55 | 70 | 55 | 70 | 0, 90 | 0, 90 | 0, 90 |
| Matrix Resin | A | A | A | A | B | A | B |
| Axial Direction Thermal Conductivity (-269°C) [w/m·k] | 0.11 | 0.10 | 0.06 | 0.05 | 0.13 | 0.13 | 0.08 |

### (Inner Container and Outer Container)

A cylindrical container, a second cylindrical container, and a refrigerant injection pipe constituting each inner container were prepared. In addition, an outer container surrounding each inner container was prepared. Table 3 illustrates configurations of the cylindrical containers, the second cylindrical containers, and the refrigerant injection pipes, and test results of the amount of evaporation of liquid helium in the insulated container.

### Example 12

A cylindrical container and a second cylindrical container in Example 12 were produced in accordance with specifications of base materials, angles, and matrix resins illustrated in Table 3. As a refrigerant injection pipe in Example 12, the refrigerant injection pipe in Example 2 was used.

### (1) Preparation of Resin for Cylindrical Container

Resin for a cylindrical container was prepared using the base resin, the curing agent, and the curing accelerator of the matrix resin A. First, 100 parts by weight of base resin, 88 parts by weight of curing agent, and 0.8 parts by weight of curing accelerator were added into a container. Subsequently, the mixture of the base resin, the curing agent, and the curing accelerator was stirred by a high-speed mixer under the condition of room temperature, 700 rpm, and 60 minutes, as a result of which the matrix resin A was obtained. Viscosity at that time was 500 mPa·s. Note that methyl ethyl ketone (MEK) was appropriately added as an organic solvent in such a manner that the viscosity of the matrix resin A became a predetermined viscosity.

### (2) Production of Cylindrical Container

### (2-1) Production of Cylindrical Portion

The alumina fibers were impregnated with the matrix resin A in such a manner that the amount of resin (wt%) of a cylindrical container after curing becomes 25 wt%. The alumina fibers impregnated with the matrix resin A were wound around a rod made of stainless steel with a diameter of 415 mm and a length of 2000 mm in a spiral manner in such a way that the alumina fibers formed an angle of 70 degrees with the axial direction of the rod. Subsequently, a polyethylene film was wrapped on the alumina fibers wound in a spiral manner and a polypropylene film was further wrapped on the polyethylene film. The alumina fibers in this state was heated at 130°C for 8 hours. After heating, the alumina fibers were cooled to room temperature and the rod made of stainless steel was removed, as a result of which the cylindrical portion was obtained.

### (2-2) Production of Upper Face Portion and Bottom Face Portion of Cylindrical Container

Glass fibers were woven in plain weave using an air-jet loom (manufactured by Toyota Industries Corporation, JAT810), as a result of which the glass cloth A was obtained. The mass per unit area of the glass cloth A was 210 g/m². Next, the same resin as the resin for a cylindrical container used in Example 5 (the matrix resin A) was prepared. After the glass cloth A was impregnated with the prepared resin, prepregs were produced in such a manner that the amount of resin (wt%) after curing under the drying condition of 100°C and 10 minutes became 30 wt%.

A plurality of prepregs was laminated in such a manner that the thickness of a laminated plate after curing becomes 30 mm and was heated and pressurized under the condition of 130°C, 0.5 MPa, and 2 hours, as a result of which the laminated plate was obtained. The laminated plate was cut in accordance with the sizes of the upper face and bottom face of the cylindrical container, as a result of which the upper face portion and the bottom face portion were obtained.

### (2-3) Joining of Upper Face Portion and Bottom Face Portion to Cylindrical Portion

The upper face portion and the bottom face portion were joined to the cylindrical portion. In order to join the upper face portion and the bottom face portion to the cylindrical portion, screw grooves were formed on the upper end side and the lower end side of the inner wall of the cylindrical portion. Further, screw threads corresponding to the screw grooves were formed on the side surfaces of the upper face portion and the bottom face portion (at locations coming into contact with the screw grooves on the cylindrical portion).

After an adhesive agent (room temperature curing epoxy resin, manufactured by Henkel Japan Ltd., Stycast 1266) was applied to joint surfaces of the cylindrical portion, the upper face portion, and the bottom face portion, the cylindrical portion and the upper face portion were joined to each other and the cylindrical portion and the bottom face portion were joined to each other. In addition, joints were sealed using an adhesive agent (room temperature curing epoxy resin, manufactured by Henkel Japan Ltd., Stycast 1266). After drying, a cylindrical container in which the thickness of the wall (wall thickness) and length of the cylindrical portion were 5 mm and 630 mm, respectively, was obtained.

### (3) Production of Second Cylindrical Container and Outer Container Matching Second Cylindrical Container

Glass fibers were woven in sateen weave using an air-jet loom (manufactured by Toyota Industries Corporation, JAT810), as a result of which the glass cloth B was obtained. The mass per unit area of the glass cloth B was 290 g/m². Next, the same resin as the resin for a cylindrical container used in Example 9 (the matrix resin B) was prepared. After the glass cloth B was impregnated with the prepared resin, prepregs were produced in such a manner that the amount of resin (wt%) after curing under the drying condition of 100°C and 10 minutes became 30 wt%.

The prepregs were stuck on the surface of a mold (a quadrangular prism shape with a height of 160 mm, a width of 183 mm, and a length of 1035 mm) copying the cylindrical portion of the second cylindrical container in such a manner that warps and wefts of the glass cloth B formed an angle of 0 degrees and an angle of 90 degrees with the axial direction of the mold, respectively, and were thereby molded into the shape of the second cylindrical container. The prepregs were stuck in layers until the thickness of the prepregs reached a thickness after drying of 6 to 8 mm. An outer container matching the cylindrical portion of the second cylindrical container was likewise produced. As the mold, a quadrangular prism shape with a height of 202 mm, a width of 225 mm, and a length of 765 mm was used.

Subsequently, a polypropylene film was wrapped on the outermost surface of the stuck prepregs. The prepregs in this state were heated at 130°C for 2 hours. Subsequently, the prepregs were cooled to room temperature and the molds were removed, as a result of which the cylindrical portion of the second cylindrical container in which the amount of resin (wt%) was 30 wt% and the outer container matching the cylindrical portion were obtained.

### Example 13

A cylindrical container in Example 13 was produced in accordance with specifications of base material, angles, and a matrix resin illustrated in Table 3. As a refrigerant injection pipe in Example 13, the refrigerant injection pipe in Example 1 was used. A second cylindrical container and an outer container matching the cylindrical portion of the second cylindrical container in Example 13 were produced using the same method as the production method of the second cylindrical container and the outer container matching the cylindrical portion of the second cylindrical container in Example 12.

The cylindrical portion constituting the cylindrical container in Example 13 has a two-layer structure in which fibers of a layer on the inner side (inner layer) are formed of alumina fibers and fibers of a layer on the outer side (outer layer) are formed of glass fibers. The cylindrical portion was produced as follows.

### (1) Preparation of Resin for Cylindrical Container

In the same manner as in Example 12, resin for a cylindrical container was prepared using the base resin, the curing agent, and the curing accelerator of the matrix resin A. First, 100 parts by weight of base resin, 88 parts by weight of curing agent, and 0.8 parts by weight of curing accelerator were added into a container. Subsequently, the mixture of the base resin, the curing agent, and the curing accelerator was stirred by a high-speed mixer under the condition of room temperature, 700 rpm, and 60 minutes, as a result of which the matrix resin A was obtained. Viscosity at that time was 500 mPa·s. Note that methyl ethyl ketone (MEK) was appropriately added as an organic solvent in such a manner that the viscosity of the matrix resin A became a predetermined viscosity.

### (2) Production of Cylindrical Container

### (2-1) Production of Cylindrical Portion

The alumina fibers were impregnated with the matrix resin A in such a manner that the amount of resin (wt%) of a cylindrical container after curing becomes 25 wt%. The alumina fibers impregnated with the matrix resin A were wound around a rod made of stainless steel with a diameter of 415 mm and a length of 2000 mm in a spiral manner in such a way that the alumina fibers formed an angle of 70 degrees with the axial direction of the rod, and the structure of the inner layer was thereby produced.

Next, the glass fibers were impregnated with the matrix resin A in such a manner that the amount of resin (wt%) of a cylindrical container after curing becomes 25 wt%. The glass fibers impregnated with the matrix resin A were wound on the surface of the inner layer formed of alumina fibers in a spiral manner in such a way that the glass fibers formed an angle of 55 degrees with the axial direction of the rod made of stainless steel. On this occasion, the glass fibers were wound in such a manner that the ratio of the thickness of the inner layer to the thickness of the outer layer was 50:50.

A polyethylene film was wrapped on the surface of the two-layer structure and a polypropylene film was further wrapped on the polyethylene film. The two-layer structure in this state was heated at 130°C for 8 hours. Subsequently, the two-layer structure was cooled to room temperature and the rod made of stainless steel was removed, as a result of which the cylindrical portion having a two-layer structure was obtained.

### Examples 14 to 15 and Comparative Example 4

As illustrated in Table 3, cylindrical containers in Examples 14 and 15 and Comparative Example 4 were produced by the same method as the production method of the cylindrical container in Example 13 except that base materials, angles, and matrix resins were changed. In addition, as a refrigerant injection pipe in Example 14, the refrigerant injection pipe in Example 2 was used. As a refrigerant injection pipe in Example 15, the refrigerant injection pipe in Example 1 was used. As a refrigerant injection pipe in Comparative Example 4, the refrigerant injection pipe in Comparative Example 1 was used. Second cylindrical containers and outer containers matching the cylindrical portions of the second cylindrical containers in Examples 14 and 15 and Comparative Example 4 were produced using the same method as the production method of the second cylindrical container and the outer container matching the cylindrical portion of the second cylindrical container in Example 12.

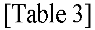

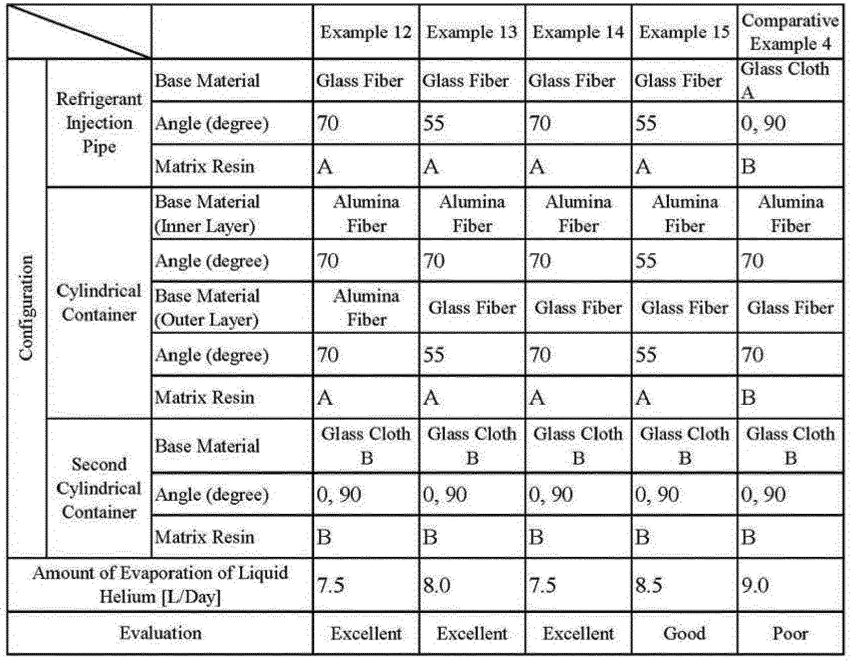

### (Production of Inner Container)

Using the refrigerant injection pipes, the cylindrical containers, and the second cylindrical containers in Examples 12 to 15 and Comparative Example 4, inner containers in Examples 12 to 15 and Comparative Example 4 were produced. First, before a refrigerant injection pipe was joined to an upper portion of a cylindrical container, flanges formed of glass/epoxy laminates were respectively attached to a joint surface of the cylindrical container and a joint surface of the refrigerant injection pipe, using an adhesive agent (room temperature curing epoxy resin, manufactured by Henkel Japan Ltd., Stycast 1266). Subsequently, an adhesive agent (room temperature curing epoxy resin, manufactured by Henkel Japan Ltd., Stycast 1266) was applied to both joint surfaces, and the refrigerant injection pipe was joined to the upper portion of the cylindrical container using screws.

Next, a hole for joining a second cylindrical container was opened on a side surface of a cylindrical portion constituting the cylindrical container. A screw groove was formed on an inner wall surface constituting the hole. Further, a screw thread corresponding to the screw groove was formed on an outer surface on the opening portion side of the second cylindrical container. The screw thread formed on the outer surface on the opening portion side of the second cylindrical container corresponds to the screw groove on the inner wall surface constituting the hole formed on the side surface of the cylindrical portion.

When the second cylindrical container was fitted into the cylindrical portion constituting the cylindrical container, the second cylindrical container was joined to the cylindrical container after an adhesive agent (room temperature curing epoxy resin, manufactured by Henkel Japan Ltd., Stycast 1266) was applied to joint surfaces thereof. In addition, joints were sealed using an adhesive agent (room temperature curing epoxy resin, manufactured by Henkel Japan Ltd., Stycast 1266). After drying, the inner container was obtained.

### (Production of Outer Container)

For the outer containers in Examples 12 to 15 and Comparative Example 4, outer containers of larger sizes than the inner containers were prepared in such a way that distances (gaps) between the inner containers and the outer containers were 30 mm or more. Specifically, a tubular body with an inner diameter of 515 mm, a wall thickness of 7 mm, and a length of 960 mm was prepared for the cylindrical portion of each outer container. A laminate with a diameter of 600 mm and a thickness of 30 mm was prepared for the upper face portion of each outer container. A laminate with a diameter of 600 mm and a thickness of 15 mm was prepared for the bottom face portion of each outer container.

A production method of the outer containers is the same method as the production method of the outer containers corresponding to the second cylindrical containers. Prepregs used at the time of producing the outer containers are the same prepregs used at the time of producing the outer containers corresponding to the second cylindrical containers. In addition, a joining method of the cylindrical portions, the upper face portions, and the bottom face portions of the outer containers and a joining method of the outer containers matching the second cylindrical containers are the same method as the joining method of the inner containers.

When thermal conductivity of the refrigerant injection pipe in Example 1 and thermal conductivity of the refrigerant injection pipe in Comparative Example 2 illustrated in Table 1 are compared with each other, it is found that, in Example 1, fibers that constitute the refrigerant injection pipe being wound in a spiral manner causes thermal conductivity in the axial direction to be low.

In addition, it is found that the thermal conductivities of all the cylindrical containers illustrated in Table 2 are low. Further, it is found that, in Example 7, fibers that constitute the cylindrical container being alumina fibers causes the thermal conductivity of the cylindrical container to be lower than the glass fibers in Example 5. Furthermore, it is found that, in Example 8, fibers that constitute the cylindrical container being wound in a spiral manner causes the thermal conductivity of the cylindrical container to be lower than the cloth in Example 11.

In addition, it is found that the insulated containers including the refrigerant injection pipes and the cylindrical containers in, for example, Examples 12 and 13 illustrated in Table 3 are capable of suppressing the amount of evaporation of liquid helium compared with the insulated container including the refrigerant injection pipe and the cylindrical container in Comparative Example 4.

From the results described above, it is found that the insulated containers of the embodiments slow the evaporation speed of refrigerant. In addition, the magnetoencephalograph and the magnetospinograph using such insulated containers are capable of reducing the number of replenishments of refrigerant, which enables long-time uninterrupted operation of the magnetoencephalograph and the magnetospinograph.

The foregoing describes some example embodiments for explanatory purposes. Although the foregoing discussion has presented specific embodiments, persons skilled in the art will recognize that changes may be made in form and detail without departing from the broader spirit and scope of the invention. Accordingly, the specification and drawings are to be regarded in an illustrative rather than a restrictive sense. This detailed description, therefore, is not to be taken in a limiting sense, and the scope of the invention is defined only by the included claims.

This application claims the benefit of Japanese Patent Application No. 2020-213175, filed on December 23, 2020.

### Reference Signs List

- 1: Circle
- 10, 40: Insulated container
- 11, 41: Inner container
- 12: Refrigerant injection pipe
- 13: Cylindrical container
- 131: Upper face portion
- 132: Cylindrical portion
- 133: Bottom face portion
- 141: Outer layer
- 142: Inner layer
- 14, 45: Outer container
- 15: Cavity
- 16: Refrigerant
- 17: Superconducting quantum interference device (SQUID)
- 18: Living body
- 19: Chair
- 21: Fiber
- 30: Magnetoencephalograph
- 42: Second cylindrical container
- 43, 44: Housing portion
- 50: Magnetospinograph

## Claims

1. An insulated container (10, 40), comprising:
an inner container (11, 41); and
an outer container (14, 45) surrounding the inner container (11, 41)with a cavity (15) interposed between the outer container (14, 45) and the inner container (11, 41), wherein
the inner container (11, 41)and the outer container (14, 45) are formed of fiber reinforced plastics in which fibers are impregnated with resin,
the inner container (11, 41) includes a bottomed cylindrical container (13) to store a refrigerant (16) and a refrigerant injection pipe (12) attached to the cylindrical container (13) and configured to inject a refrigerant (16) into the cylindrical container (13), and
fibers constituting the refrigerant injection pipe (12) are wound in a spiral manner in an axial direction of the refrigerant injection pipe (12).

2. The insulated container (10, 40) according to claim 1, wherein
fibers constituting the refrigerant injection pipe (12) are wound in a spiral manner in such a way as to form an angle of 50 to 89 degrees with an axial direction of the refrigerant injection pipe (12).

3. The insulated container (10, 40) according to claim 1 or 2, wherein
fibers constituting the refrigerant injection pipe (12) are formed of at least one type of fiber selected from a group including glass fiber, alumina fiber, and carbon fiber.

4. The insulated container (10, 40) according to any one of claims 1 to 3,
wherein
an amount of resin in fiber reinforced plastics that constitute the refrigerant injection pipe (12) is 15 to 40 wt% of entire weight of the fiber reinforced plastics.

5. The insulated container (10, 40) according to any one of claims 1 to 4,
wherein
fibers constituting the cylindrical container (13) are formed of at least one type of fiber selected from a group including glass fiber, alumina fiber, and carbon fiber.

6. The insulated container (10, 40) according to any one of claims 1 to 5,
wherein
the resin includes epoxy resin.

7. A magnetoencephalograph (30), comprising:
a superconducting quantum interference device (17) to detect a magnetic field; and
the insulated container (10, 40) according to any one of claims 1 to 6, wherein
the superconducting quantum interference device (17) is housed in the cylindrical container (13) while being dipped in a refrigerant (16) stored in the cylindrical container (13).

8. The insulated container (10, 40) according to any one of claims 1 to 6,
wherein
the inner container (11, 41) further includes a second cylindrical container (42),
each of the cylindrical container (11, 41) and the second cylindrical container (42) includes a housing portion (43, 44) to store a refrigerant (16) inside the housing portion (43, 44),
the second cylindrical container (42) is fixed to the cylindrical container (13) while the housing portion (44) of the second cylindrical container (42) and the housing portion (43) of the cylindrical container (11, 41) communicate with each other, and
the second cylindrical container (42) is formed of fiber reinforced plastics in which fibers are impregnated with resin.

9. The insulated container (10, 40) according to claim 8, wherein
fibers constituting the second cylindrical container (42) are formed of at least one type of fiber selected from a group including glass fiber, alumina fiber, and carbon fiber.

10. The insulated container (10, 40) according to claim 8 or 9, wherein
resin constituting the second cylindrical container (42) includes epoxy resin.

11. A magnetospinograph (50), comprising:
a superconducting quantum interference device (17) to detect a magnetic field; and
the insulated container (10, 40) according to any one of claims 8 to 10, wherein
the superconducting quantum interference device (17) is housed in the housing portion (44) of the second cylindrical container (42) while being dipped in a refrigerant (16) stored in the housing portion (44) of the second cylindrical container (42).

## Patentansprüche

1. Isolierter Behälter (10, 40), umfassend:
einen Innenbehälter (11, 41); und
einen Außenbehälter (14, 45), der den Innenbehälter (11, 41) umgibt, mit einem Hohlraum (15), der zwischen dem Außenbehälter (14, 45) und dem Innenbehälter (11, 41) eingeschoben ist, wobei
der Innenbehälter (11, 41) und der Außenbehälter (14, 45) aus faserverstärkten Kunststoffen geformt sind, in denen Fasern mit Harz imprägniert sind,
wobei der Innenbehälter (11, 41) einen mit einem Boden versehenen zylindrischen Behälter (13), um ein Kältemittel (16) zu speichern, und ein Kältemitteleinspritzrohr (12) einschließt, das an dem zylindrischen Behälter (13) angeheftet und konfiguriert ist, um ein Kältemittel (16) in den zylindrischen Behälter (13) einzuspritzen, und
Fasern, die das Kältemitteleinspritzrohr (12) bilden, spiralförmig in einer Achsrichtung des Kältemitteleinspritzrohrs (12) gewickelt sind.

2. Isolierter Behälter (10, 40) nach Anspruch 1, wobei
Fasern, die das Kältemitteleinspritzrohr (12) bilden, derart spiralförmig gewickelt sind, um einen Winkel von 50 bis 89 Grad mit einer Achsrichtung des Kältemitteleinspritzrohrs (12) zu formen.

3. Isolierter Behälter (10, 40) nach Anspruch 1 oder 2, wobei
Fasern, die das Kältemitteleinspritzrohr (12) bilden, aus mindestens einem Fasertyp geformt sind, ausgewählt aus einer Gruppe einschließend Glasfaser, Aluminiumoxidfaser und Kohlefaser.

4. Isolierter Behälter (10, 40) nach einem der Ansprüche 1 bis 3, wobei
eine Harzmenge in faserverstärkten Kunststoffen, die das Kältemitteleinspritzrohr (12) bilden, 15 bis 40 % vom Gesamtgewicht der faserverstärkten Kunststoffe ist.

5. Isolierter Behälter (10, 40) nach einem der Ansprüche 1 bis 4, wobei
Fasern, die den zylindrischen Behälter (13) bilden, aus mindestens einem Fasertyp geformt sind, ausgewählt aus einer Gruppe einschließend Glasfaser, Aluminiumoxidfaser und Kohlefaser .

6. Isolierter Behälter (10, 40) nach einem der Ansprüche 1 bis 5, wobei
das Harz Epoxidharz einschließt.

7. Magnetoenzephalograph (30), umfassend:
eine supraleitende Quanteninterferenzvorrichtung (17), um ein Magnetfeld zu detektieren; und
den isolierten Behälter (10, 40) nach einem der Ansprüche 1 bis 6, wobei
die supraleitende Quanteninterferenzvorrichtung (17) in dem zylindrischen Behälter (13) aufgenommen ist, während sie in ein Kältemittel (16) getaucht wird, das in dem zylindrischen Behälter (13) gespeichert ist.

8. Isolierter Behälter (10, 40) nach einem der Ansprüche 1 bis 6, wobei
der Innenbehälter (11, 41) ferner einen zweiten zylindrischen Behälter (42) einschließt,
wobei jeder der zylindrischen Behälter (11, 41) und der zweite zylindrische Behälter (42) einen Aufnahmeabschnitt (43, 44) einschließt, um ein Kältemittel (16) im Inneren des Aufnahmeabschnitts (43, 44) zu speichern,
wobei der zweite zylindrische Behälter (42) an dem zylindrischen Behälter (13) befestigt ist, während der Aufnahmeabschnitt (44) des zweiten zylindrischen Behälters (42) und der Aufnahmeabschnitt (43) des zylindrischen Behälters (11, 41) miteinander in Verbindung stehen, und
der zweite zylindrische Behälter (42) aus faserverstärkten Kunststoffen geformt ist, in denen Fasern mit Harz imprägniert sind.

9. Isolierter Behälter (10, 40) nach Anspruch 8, wobei
Fasern, die den zweiten zylindrischen Behälter (42) bilden, aus mindestens einem Fasertyp geformt sind, ausgewählt aus einer Gruppe einschließend Glasfaser, Aluminiumoxidfaser und Kohlefaser.

10. Isolierter Behälter (10, 40) nach Anspruch 8 oder 9, wobei
Harz, das den zweiten zylindrischen Behälter (42) bildet, Epoxidharz einschließt.

11. Magnetospinograph (50), umfassend:
eine supraleitende Quanteninterferenzvorrichtung (17), um ein Magnetfeld zu detektieren; und
den isolierten Behälter (10, 40) nach einem der Ansprüche 8 bis 10, wobei
die supraleitende Quanteninterferenzvorrichtung (17) in dem Aufnahmeabschnitt (44) des zweiten zylindrischen Behälters (42) aufgenommen ist, während sie in ein Kältemittel (16) getaucht wird, das in dem Aufnahmeabschnitt (44) des zweiten zylindrischen Behälters (42) gespeichert ist.

## Revendications

1. Récipient isolé (10, 40), comprenant :
un récipient intérieur (11, 41) ; et
un récipient extérieur (14, 45) entourant le récipient intérieur (11, 41) avec une cavité (15) intercalée entre le récipient extérieur (14, 45) et le récipient intérieur (11, 41), dans lequel
le récipient intérieur (11, 41) et le récipient extérieur (14, 45) sont formés de plastique renforcé en fibres dans lequel des fibres sont imprégnées avec de la résine,
le récipient intérieur (11, 41) inclut un récipient cylindrique (13) muni d'un fond pour stocker un réfrigérant (16) et un tuyau d'injection de réfrigérant (12) fixé sur le récipient cylindrique (13) et configuré pour injecter un réfrigérant (16) dans le récipient cylindrique (13), et
des fibres constituant le tuyau d'injection de réfrigérant (12) sont enroulées en spirale dans une direction axiale du tuyau d'injection de réfrigérant (12).

2. Récipient isolé (10, 40) selon la revendication 1, dans lequel
les fibres constituant le tuyau d'injection de réfrigérant (12) sont enroulées en spirale de façon à former un angle de 50 à 89 degrés avec une direction axiale du tuyau d'injection de réfrigérant (12).

3. Récipient isolé (10, 40) selon la revendication 1 ou 2, dans lequel
les fibres constituant le tuyau d'injection de réfrigérant (12) sont formées d'au moins un type de fibre sélectionné dans un groupe incluant la fibre de verre, la fibre d'alumine, et la fibre de carbone.

4. Récipient isolé (10, 40) selon l'une quelconque des revendications 1 à 3, dans lequel
une quantité de résine dans le plastique renforcé en fibres qui constitue le tuyau d'injection de réfrigérant (12) est de 15 à 40 % en poids du poids entier du plastique renforcé en fibres.

5. Récipient isolé (10, 40) selon l'une quelconque des revendications 1 à 4, dans lequel
les fibres constituant le récipient cylindrique (13) sont formées d'au moins un type de fibre sélectionné depuis un groupe incluant la fibre de verre, la fibre d'alumine, et la fibre de carbone.

6. Récipient isolé (10, 40) selon l'une quelconque des revendications 1 à 5, dans lequel
la résine inclut de la résine époxy.

7. Magnétoencéphalographe (30), comprenant :
un dispositif d'interférence quantique supraconducteur (17) pour détecter un champ magnétique ; et
le récipient isolé (10, 40) selon l'une quelconque des revendications 1 à 6, dans lequel
le dispositif d'interférence quantique supraconducteur (17) est logé dans le récipient cylindrique (13) tout en étant plongé dans un réfrigérant (16) stocké dans le récipient cylindrique (13).

8. Récipient isolé (10, 40) selon l'une quelconque des revendications 1 à 6, dans lequel
le récipient intérieur (11, 41) inclut en outre un second récipient cylindrique (42),
chacun du récipient cylindrique (11, 41) et du second récipient cylindrique (42) inclut une partie de logement (43, 44) pour stocker un réfrigérant (16) à l'intérieur de la partie de logement (43, 44),
le second récipient cylindrique (42) est fixé sur le récipient cylindrique (13) alors que la partie de logement (44) du second récipient cylindrique (42) et la partie de logement (43) du récipient cylindrique (11, 41) communiquent ensemble, et
le second récipient cylindrique (42) est formé de plastique renforcé en fibres dans lequel les fibres sont imprégnées avec de la résine.

9. Récipient isolé (10, 40) selon la revendication 8, dans lequel les fibres constituant le second récipient cylindrique (42) sont formées d'au moins un type de fibre sélectionné dans un groupe incluant la fibre de verre, la fibre d'alumine, et la fibre de carbone.

10. Récipient isolé (10, 40) selon la revendication 8 ou 9, dans lequel la résine constituant le second récipient cylindrique (42) inclut de la résine époxy.

11. Magnétospinographe (50), comprenant :
un dispositif d'interférence quantique supraconducteur (17) pour détecter un champ magnétique ; et
le récipient isolé (10, 40) selon l'une quelconque des revendications 8 à 10, dans lequel
le dispositif d'interférence quantique supraconducteur (17) est logé dans la partie de logement (44) du second récipient cylindrique (42) tout en étant plongé dans un réfrigérant (16) stocké dans la partie de logement (44) du second récipient cylindrique (42).
